# EUROPEAN PATENT SPECIFICATION

(11) **EP 2 169 097 B1**
(45) Date of publication and mention of the grant of the patent: **20.04.2011**
(21) Application number: 09011183.2
(22) Date of filing: 31.08.2009
(51) Int. Cl.: D01H 13/14, D01H 13/22, D01H 13/26, B65H 63/06, G01N 21/89, G01N 33/36

(54) **Foreign substance detecting device and method in textile machine**
Fremdstoff erfassungsvorrichtung und -verfahren in textilmaschine
Dispositif et méthode de détection de matières contaminentes pour une machine textile

(30) Priority: 25.09.2008 JP 2008246382
(43) Date of publication of application: 31.03.2010
(73) Proprietor: Murata Machinery, Ltd., Minami-ku Kyoto-shi Kyoto 601-8326 (JP)
(72) Inventor: Nakatani, Masatoshi, Kyoto-shi Kyoto 612-8686 (JP)
(74) Representative: Schoppe, Fritz

(56) References cited:
- EP-A- 1 574 607
- EP-A- 1 808 690
- WO-A-01/92875
- JP-A- 2007 291 544
- US-A- 5 875 419

## Description

### BACKGROUND OF THE INVENTION

### 1. Field of the Invention

The present invention primarily relates to a foreign substance detecting device for a yarn used in a textile machine or the like.

### 2. Description of the Related Art

In a textile machine such as a spinning machine, a foreign substance detecting device for detecting a foreign substance in a traveling yarn is known. Such a foreign substance detecting device emits light to the traveling yarn, measures a change in an amount of received light which has been reflected, and thus, detects a foreign substance. In a textile machine including such a foreign substance detecting device, when a foreign substance is detected by the foreign substance detecting device, a portion including the foreign substance is cut and removed, and a yarn splicing operation is performed.

Some foreign substances, such as a small and unnoticeable foreign substance and a foreign substance that becomes unnoticeable by being bleached in a subsequent process, may not be a problem even when being included in a yarn. However, when determining a foreign substance based only on the change in the amount of received light, even a portion including such a problem-free foreign substance is cut and removed. As a result, a production efficiency of the entire textile machine is reduced. In view of the above point, Japanese Unexamined Patent Application Publication No. 2007-291544 (Patent Document 1) discloses a foreign substance detecting device that can distinguish and detect a trash (which becomes unnoticeable by being bleached and, accordingly, does not need to be removed) and a colored yarn (which needs to be removed) by analyzing an amount of the received light reflected by a yarn.

The "trash" refers to a portion of a yarn contaminated with a cotton trash of a sliver or a fiber cluster or the like that are included in a sliver. The "colored yarn" refers to a portion of a yarn including a black or dark-colored fiber etc. that has mixed into a sliver during transportation. Such foreign substances are generally deeper in color than fibers of a spun yarn and, accordingly, reduce reflected light. The colored yarn does not reduce the reflected light as much as the trash does. Therefore, a portion including the trash may be represented as "dark", and a portion including the colored yarn may be represented as "slightly dark". When measuring the amount of the received light reflected by the traveling yarn, if the yarn is detected to include a dark and short portion, the foreign substance detecting device disclosed in the Patent Document 1 determines that such a yarn portion is a trash, and if the yarn is detected to include a slightly-dark and long portion, the foreign substance detecting device determines that such a yarn portion is a colored yarn.

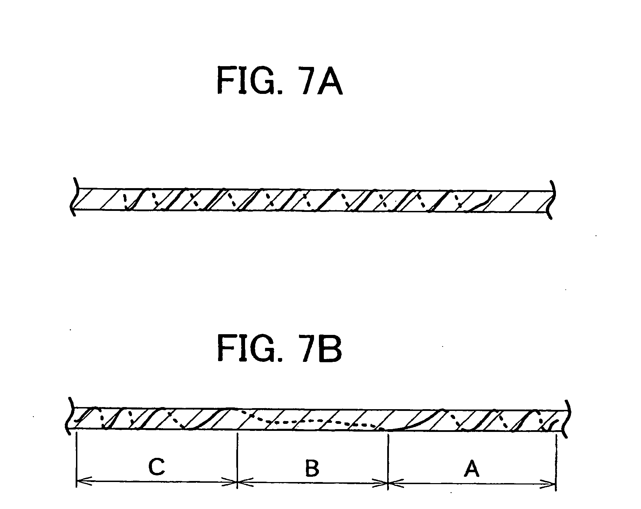

When a dark-colored fiber or the like mixed in a sliver is twisted with a fiber bundle by a spinning machine, a spun yarn including a colored yarn is generally formed with the dark-colored fiber or the like being spirally wound around a surface of the spun yarn as illustrated in Fig. 7A. A section in which the colored yarn is in spirals reduces a reflection of the emitted light, and accordingly, such section is observed as a "slightly dark" portion. Therefore, a presence of the colored yarn can be detected by the foreign substance detecting device disclosed in the Patent Document 1.

However, as illustrated in a section B of Fig. 7B, a portion of the colored yarn may be linear. In such a colored yarn, the section B hardly reduces the reflected light as compared with sections A and C in which the colored yarn is in spirals. Moreover, when the linear portion of the colored yarn indicated by dashed line in the section B is hidden by the spun yarn and invisible, the linear portion cannot be detected by the reflected light. Therefore, when the light reflected from the section B of the spun yarn of Fig. 7B is measured, a value that is substantially similar to an amount of light received from a yarn having no foreign substance is detected.

As described above, the colored yarn having the linear portion could not be detected as the foreign substance by the foreign substance detecting device disclosed in the Patent Document 1. This is because the foreign substance detecting device disclosed in the Patent Document 1 detects the "dark and short" foreign substance (trash) and the "slightly-dark and long" foreign substance (colored yarn).

In other words, since the sections A and C of Fig. 7B are "slightly dark and short" but are not as "dark" as the trash, the sections A and C are not detected as the "dark and short" foreign substance. The sections A, B, and C in combination may be a "long" section, however, since the light reflected from the section B is bright, the entire section is too bright to be represented as "slightly dark", and accordingly, such entire section is not detected as the "slightly-dark and long" foreign substance.

The "slightly-dark and short" portion such as the sections A and C is not noticeable to such a degree that the individual portion does not need to be removed. Therefore, such a portion is not detected as the foreign substance. However, for example, a dark-colored fiber with a length of 5 cm or longer including the consecutive sections A, B, and C may be highly noticeable. In the colored yarn illustrated in Fig. 7B, it can be visually checked from another angle that the linear portion and the spiral portions are consecutive. Accordingly, when cloth is woven with a yarn in which such a colored yarn is remaining, the colored yarn portion is highly noticeable. As a result, a commodity value of the cloth is reduced.

Accordingly, a foreign substance detecting device that can detect such colored yarns has been desired.

### SUMMARY OF THE INVENTION

In order to overcome the problems described above, preferred embodiments of the present invention provide a foreign substance detecting device that can reliably detect a colored yarn.

According to a first aspect of the present invention, a foreign substance detecting device includes a light emitting section, a light receiving section, a measuring section, a received-light value detecting section, a storage section, a calculating section, and a determining section. The light emitting section emits light to a traveling yarn. The light receiving section receives light which is a reflection of the light emitted from the light emitting section and reflected by the yarn. The measuring section measures an amount of the light received by the light receiving section for each prescribed yarn length interval. The received-light value detecting section obtains a received-light value in accordance with the measured value measured by the measuring section. The storage section stores a plurality of received-light values obtained by the received-light value detecting section. The calculating section calculates a sum of a newest received-light value, which has been obtained by the received-light value detecting section, and a received-light value that is stored in the storage section after being calculated in accordance with a measured value measured at a position located downstream for a prescribed length than the yarn length interval from the measurement position of the measured value corresponding to the newest received-light value. The determining section determines a presence or an absence of a foreign substance in accordance with the sum calculated by the calculating section.

In other words, since the sum of the received-light values is calculated at prescribed intervals, and the presence or the absence of the foreign substance is determined in accordance with the sum, a foreign substance determination can be performed by skipping a portion of the prescribed length. Thus, even the colored yarn that is linear at a middle portion can be detected as the foreign substance without being influenced by a measured value of the middle portion. Accordingly, the colored yarn can be accurately detected.

Further, the foreign substance detecting device preferably includes a minimum value detecting section. The minimum value detecting section obtains a minimum received-light value from the plurality of received-light values, which have been stored in the storage section after being obtained in accordance with measured values measured within a range of a prescribed second length located downstream for a prescribed first length than the yarn length interval from the measurement position of the measured value corresponding to the newest received-light value. The calculating section calculates a sum of the newest received-light value and the minimum received-light value.

In other words, by having the first length interval between the newest received-light value and the minimum received-light value, the sum that has skipped the linear portion of the colored yarn can be calculated. Moreover, since the past received-light value obtained within the range of the second length having a certain length is considered, the colored yarn can be detected while taking into account a fact that the length of the middle portion in which the colored yarn is linear varies for each colored yarn. Further, since the sum of the past minimum received-light value and the newest received-light value is calculated, it is not necessary to calculate the sum of the newest received-light value and every past received-light values obtained within the range of the second length in order to determine the presence or the absence of the foreign substance. As a result, a calculation efficiency can be improved.

In the foreign substance detecting device, it is preferable that the minimum value detecting section stores the obtained minimum received-light value, and when the measurement position of the measured value corresponding to the minimum received-light value falls outside the range of the second length, the minimum value detecting section newly obtains a minimum received-light value in the range of the second length and updates the minimum received-light value stored in the storage section. Accordingly, in order to obtain the minimum value, it is not necessary to compare all of the received-light values corresponding to the measurement positions located within the range of the second length each time. As a result, the calculation efficiency can be improved.

In the foreign substance detecting device, it is preferable that when a received-light value, which has been obtained in accordance with a measured value measured at a measurement position newly entering into the range of the second length, is smaller than the stored minimum received-light value, the minimum value detecting section updates the minimum received-light value stored in the storage section. Thus, the minimum received-light value stored in the storage section can be easily updated. As a result, the calculation efficiency can be further improved.

In the foreign substance detecting device, it is preferable that the received-light value detecting section obtains the received-light value by considering a measured value measured in a range located downstream for a prescribed third length from the measurement position of the measured value corresponding to the received-light value. By obtaining the received-light value by considering the measured value measured for the range having a certain length, detection accuracy can be improved while minimizing noise influence.

In the foreign substance detecting device, it is preferable that the determining section determines the presence or the absence of the foreign substance by comparing the sum calculated by the calculating section and a prescribed threshold value. Thus, the foreign substance can be detected by a simple process of comparing the sum and the threshold value.

A second aspect of the present invention provides a textile machine including the above-described foreign substance detecting device. In the textile machine having the above structure, a colored yarn that is linear at a middle portion can be detected. Therefore, a package quality can be improved.

A third aspect of the present invention provides a following foreign substance detecting method. That is, the foreign substance detecting method includes a measuring step, a received-light value detecting step, a storing step, a calculating step, and a comparing step. The measuring step emits light to a traveling yarn, receives light which is a reflection of the emitted light reflected by the yarn, and measures an amount of received light for each prescribed yarn length interval. The received-light value detecting step obtains a received-light value in accordance with a measured value of the amount of the received light. The storing step stores the received-light value in a storage section. The calculating step calculates a sum of a newest received-light value and a received-light value which has been stored in the storage section after being obtained in accordance with a measured value measured at a position located downstream for a prescribed length than the yarn length interval from the measurement position of a measured value corresponding to the newest received-light value. The comparing step detects a foreign substance by comparing the sum and a prescribed threshold value.

Since the above method calculates the sum of the received-light values at prescribed intervals and determines the presence or the absence of the foreign substance in accordance with the sum, the foreign substance determination can be performed by skipping a portion of the prescribed length. Thus, even the colored yarn that is linear at the middle portion can be detected as the foreign substance without being influenced from the measured value of the middle portion. Accordingly, the colored yarn can be detected more accurately.

### BRIEF DESCRIPTION OF THE DRAWINGS

Fig. 1 is a front view illustrating an entire structure of a spinning machine according to an embodiment of the present invention.

Fig. 2 is a block diagram illustrating a flow of a control signal or the like in the spinning machine.

Fig. 3 is a block diagram illustrating a functional structure of a yarn clearer and a schematic planar cross-sectional view of a clearer head according to an embodiment of the present invention.

Fig. 4 schematically illustrates data of a light receiving rate stored in a first storage section.

Fig. 5 schematically illustrates a light receiving rate sum stored in a second storage section.

Fig. 6 is a flowchart of a foreign substance detecting method according to an embodiment of the present invention.

Fig. 7A illustrates an appearance of a colored yarn. Fig. 7B illustrates an appearance of a colored yarn that is linear at a middle portion.

### DETAILED DESCRIPTION OF PREFERRED EMBODIMENTS

A spinning machine as a textile machine according to an embodiment of the present invention will be described with reference to the drawings. In the description, "upstream" and "downstream" respectively refer to upstream and downstream in a direction in which a yarn travels during a spinning operation.

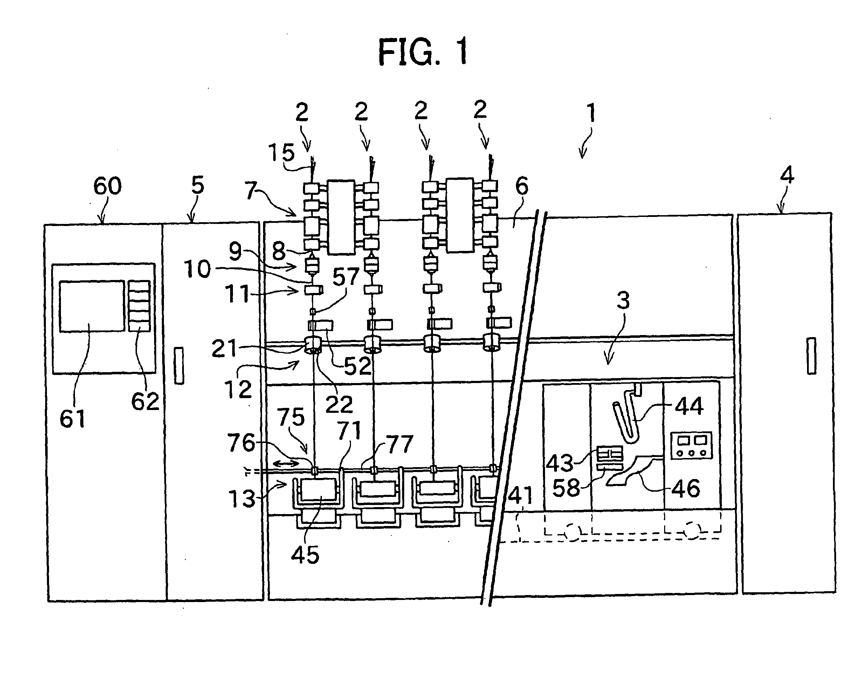

A spinning machine 1 as the textile machine illustrated in Fig. 1 includes a plurality of aligned spinning units 2, a machine control device 60, a yarn splicing cart 3, a blower box 4, and a motor box 5. The spinning machine 1 further includes a not-illustrated automatic doffing device.

The machine control device 60 collectively manages each constituent element of the spinning machine 1. The machine control device 60 includes a monitor 61 and input keys 62. The machine control device 60 can transmit and/or receive information to and/or from each of the constituent elements of the spinning machine 1.

For example, the monitor 61 can display a graph of a production efficiency of each of the spinning units 2, and a spinning unit 2 in which an error has occurred. The monitor 61 can also display statistical information on the entire spinning machine 1. An operator can perform a prescribed operation using the input keys 62 to change a setting of a certain spinning unit 2 or collectively change settings of all of the spinning units 2. (Such setting change is performed by transmitting setting data from the machine control device 60 to a unit control section 73 which is to be described later.) The machine control device 60 can also control and manage, for example, the yarn splicing cart 3 and the not-illustrated automatic doffing device in addition to the spinning units 2.

As illustrated in Fig. 1, each of the spinning units 2 forms a package 45 from a sliver 15. Each of the spinning units 2 includes a draft device 7, a spinning device 9, a yarn feeding device 11, a yarn slack eliminating device 12, and a winding device 13, which are arranged in this order from the upstream to the downstream. The draft device 7 is provided in the vicinity of an upper end of a frame 6 of the spinning machine 1. The spinning device 9 spins a fiber bundle 8 fed from the draft device 7. A spun yarn 10 discharged from the spinning device 9 is fed by the yarn feeding device 11, and after passing through a yarn clearer 52 to be described later, the spun yarn 10 is wound by the winding device 13 into the package 45.

The yarn splicing cart 3 can travel in a direction in which the spinning units 2 are aligned. At the time of yarn cut (to be described later), the yarn splicing cart 3 travels to a position of the spinning unit 2 that needs a yarn splicing operation, and splices an upper yarn from the spinning device 9 and a lower yarn from the winding device 13.

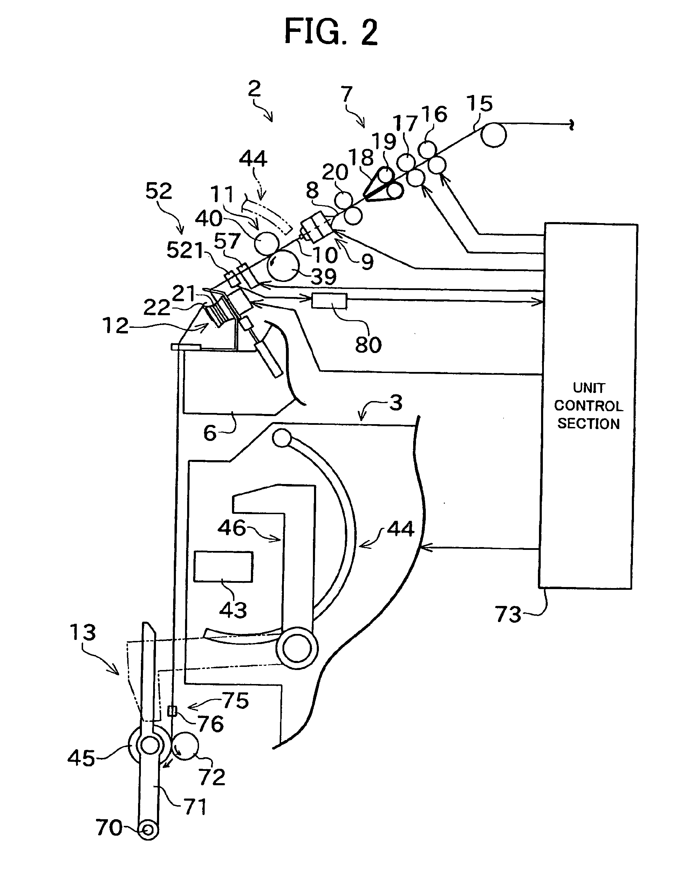

Next, with reference to Fig. 2, a structure of each of the spinning units 2 will be described in detail. The spinning machine 1 includes a unit control section 73 for controlling each of the spinning units 2. The unit control section 73 controls each constituent element of the spinning unit 2 based on a control signal transmitted from the machine control device 60 and on a signal detected by various sensors of each of the spinning units 2, or the like. The unit control section 73 can transmit information relating to each of the spinning units 2 to the machine control device 60.

The draft device 7 drafts the sliver 15 into the fiber bundle 8. The draft device 7 includes a back roller 16, a third roller 17, a middle roller 19, and a front roller 20. Further, an apron belt 18 is wound around the middle roller 19.

Although a detailed structure of the spinning device 9 is not illustrated in the drawings, the spinning device 9 according to the present embodiment is a pneumatic type which uses a whirling airflow to apply twists to the fiber bundle 8 and to form the spun yarn 10.

The yarn feeding device 11 includes a delivery roller 39 and a nip roller 40. The delivery roller 39 is supported by the frame 6 of the spinning machine 1. The nip roller 40 is in contact with the delivery roller 39. Under a state in which the spun yarn 10 discharged from the spinning device 9 is nipped between the delivery roller 39 and the nip roller 40, the delivery roller 39 is rotationally driven by a not-illustrated electric motor to feed the spun yarn 10 to the winding device 13.

The yarn clearer (foreign substance detecting device) 52 is arranged at a position that is located on a front surface side of the frame 6 of the spinning machine 1 and on a slightly downstream side of the yarn feeding device 11. The yarn clearer 52 primarily includes a clearer head 521 and an analyzer 80. The spun yarn 10, which is spun by the spinning device 9, passes through the clearer head 521 before being wound by the winding device 13. The yarn clearer 52 monitors a thickness and a foreign substance or the like of the traveling spun yarn 10. When a yarn defect of the spun yarn 10 is detected, the yarn clearer 52 transmits a yarn defect detection signal to the unit control section 73. A cutter 57 is provided in the vicinity of the yarn clearer 52 for cutting the spun yarn 10 when a yarn defect is detected. Further, a detailed structure of the yarn clearer 52 will be described later.

The yarn slack eliminating device 12 can eliminate a slack of the spun yarn 10 between the spinning device 9 and the winding device 13 (i.e., between the spinning device 9 and a splicer 43 to be described later) and apply an appropriate tension to the spun yarn 10. The yarn slack eliminating device 12 primarily includes a slack eliminating roller 21 and a yarn hooking member 22. Although a description of a detailed structure of the yarn slack eliminating device 12 is omitted, the yarn slack eliminating device 12 accumulates the spun yarn 10 by winding the spun yarn 10 around an outer surface of the slack eliminating roller 21, and functions the accumulated spun yarn 10 as a buffer. Accordingly, the yarn slack eliminating device 12 prevents the yarn slack by absorbing tension changes in the spun yarn 10.

Next, the winding device 13 will be described. The winding device 13 includes a cradle arm 71 that is supported on a supporting shaft 70 in a manner that the cradle arm 71 can swing around the supporting shaft 70. The cradle arm 71 supports a bobbin, around which the spun yarn 10 is wound, in a manner that the bobbin can be rotated. The winding device 13 includes a winding drum 72 and a traverse device 75.

The winding drum 72 is driven in contact with an outer peripheral surface of the bobbin or the package 45, which is formed by winding the spun yarn 10 around the bobbin. The traverse device 75 includes a traverse guide 76 that can be engaged with the spun yarn 10. The traverse guide 76 is fixed on a traverse rod 77 that is horizontally arranged across the plurality of spinning units 2. By driving the winding drum 72 by a not-illustrated electric motor while reciprocating the traverse rod 77 by a not-illustrated driving mechanism, the package 45 that is in contact with the winding drum 72 can be rotated, and the spun yarn 10 can be traversed and wound.

Next, the yarn splicing cart 3 will be described. As illustrated in Figs. 1 and 2, the yarn splicing cart 3 includes the splicer (a yarn splicing device) 43, a suction pipe 44, a suction mouth 46, and a lower yarn detecting sensor 58. The yarn splicing cart 3 travels on a rail 41 provided on the frame 6 of the spinning machine 1. When a yarn breakage or a yarn cut occurs in a certain spinning unit 2, the yarn splicing cart 3 travels to and stops at such spinning unit 2, and performs a yarn splicing operation.

The yarn splicing operation of the yarn splicing cart 3 will now be described in detail. As described above, when the yarn clearer 52 detects a yarn defect in the spun yarn 10, the yarn defect detection signal is transmitted to the unit control section 73. When the unit control section 73 receives the yarn defect detection signal, the unit control section 73 immediately cuts the yarn by the cutter 57, and stops the draft device 7, the spinning device 9, and the winding device 13 or the like. Then, the unit control section 73 controls the yarn splicing cart 3 to travel to the front of the spinning unit 2.

Then, the unit control section 73 re-drives the draft device 7 and the spinning device 9 or the like of the spinning unit 2, and starts catching of yarn ends by the suction pipe 44 and the suction mouth 46 of the yarn splicing cart 3. While vertically swinging around a shaft, the suction pipe 44 sucks and catches the yarn end of the upper yarn discharged from the spinning device 9, and then guides the yarn end to the splicer 43. At almost the same time, while vertically swinging around a shaft, the suction mouth 46 sucks and catches a yarn end of the lower yarn from the package 45, which is rotatably supported by the winding device 13, and then guides the yarn end to the splicer 43.

When the upper yarn and the lower yarn are guided to the splicer 43, the splicer 43 performs a yarn splicing operation on the guided yarn ends. Although a description on a detailed structure of the splicer 43 is omitted, for example, the yarn splicing operation may be performed through a method of twisting the yarn ends together using fluid such as compressed airflow. When the yarn splicing operation is completed, a winding operation by the winding device 13 is resumed.

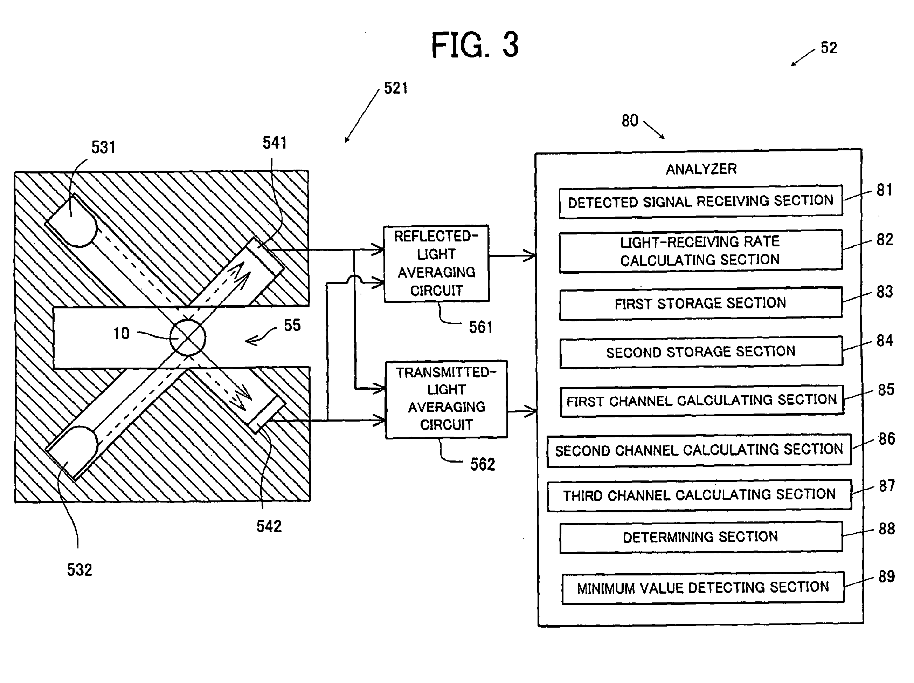

Next, with reference to Fig. 3, a structure of the clearer head 521 of the yarn clearer 52 will be described. The clearer head 521 includes light emitting sections 531, 532 composed of a light emitting element such as Light Emitting Diodes (LED), light receiving sections 541, 542 composed of a photoelectric conversion element, a reflected-light averaging circuit 561, and a transmitted-light averaging circuit 562. Moreover, a yarn passage 55 is formed in the clearer head 521. The spun yarn 10 travels through the yarn passage 55.

The light emitting sections 531, 532 are arranged in a manner that the light emitting sections 531, 532 emit light to the spun yarn 10 from respective directions that are different from each other. By emitting light alternately from the light emitting sections 531, 532 for each prescribed yarn length or for each prescribed period of time, the spun yarn 10 is monitored from two different directions. (Fig. 3 illustrates as if the light emitting sections 531, 532 simultaneously emit light. In reality, however, only one of the light emitting sections 531, 532 emits light at one time.) Thus, an effect of externally intruding light and/or an effect of other light sources are eased, and a foreign substance can be reliably detected.

The light emitted from the light emitting section 531 or 532 to the spun yarn 10 is transmitted through the spun yarn 10 (solid arrow of Fig. 3) and is partially reflected by the spun yarn 10 (dashed arrow of Fig. 3). As illustrated in Fig. 3, when the light is emitted from the light emitting section 531, the light receiving section 542 receives the transmitted light, and the light receiving section 541 receives the reflected light. When the light is emitted from the light emitting section 532, the light receiving section 541 receives the transmitted light, and the light receiving section 542 receives the reflected light. Each of the light receiving sections 541, 542 outputs a voltage value that is proportional to an amount of the received light. By measuring the voltage values of the light receiving sections 541, 542, an amount of the transmitted light and an amount of the reflected light which are received by the light receiving sections 542, 542 can be detected.

As described above, since the light emitting sections 531, 532 alternately emit light, the light receiving sections 541, 542 alternately receive the reflected light and the transmitted light. The voltage value, which is output when the light receiving sections 541, 542 receive the reflected light or the transmitted light, is divided and input to the reflected-light averaging circuit 561 or the transmitted-light averaging circuit 562 via a not-illustrated switching element.

The reflected-light averaging circuit 561 receives, as the input, a voltage value that is output when the light receiving section 541 receives the reflected light from the light emitting section 531, and a voltage value that is output when the light receiving section 542 receives the reflected light from the light emitting section 532. Then, the reflected-light averaging circuit 561 outputs an average value of the two voltage values as a detected voltage value of the reflected light. The transmitted-light averaging circuit 562 receives, as the input, the voltage value that is output when the light receiving section 541 receives the transmitted light from the light emitting section 532 and the voltage value that is output when the light receiving section 542 receives the transmitted light from the light emitting section 531. Then, the transmitted-light averaging circuit 562 outputs an average value of the two voltage values as a detected voltage of the transmitted light.

In the above configuration, when the spun yarn 10 has a thick portion, the received amount of the transmitted light decreases, and when the spun yarn 10 has a thin portion, the received amount of the transmitted light increases. In other words, the received amount of the transmitted light changes depending on the thickness of the spun yarn 10. Therefore, the thickness of the spun yarn 10 can be detected by measuring the detected voltage of the transmitted light. When the spun yarn 10 includes a dark foreign substance, the received amount of the reflected light decreases. When the spun yarn 10 includes a bright foreign substance, the received amount of the reflected light increases. In other words, the received amount of the reflected light changes depending on a presence of a foreign substance. Therefore, the foreign substance in the spun yarn 10 can be detected by measuring the detected voltage of the reflected light.

Next, a structure of the analyzer 80 of the yarn clearer 52 will be described. The analyzer 80 receives the detected voltage of the reflected light and the detected voltage of the transmitted light from the clearer head 521, and analyzes the received detected voltages in order to detect a foreign substance. When a foreign substance to be removed is detected, the analyzer 80 transmits the yarn defect detection signal to the unit control section 73.

For example, the analyzer 80 is provided as a micro controller, and includes a not-illustrated Central Processing Unit (CPU), a Random Access memory (RAM), a Read Only Memory (ROM), and an Input-and-Output (I/O), or the like. The ROM stores various programs. When such programs are executed, the CPU, the RAM, the ROM, and the I/O together control the analyzer 80 to function as a later-described detected signal receiving section 81, a light-receiving rate calculating section 82, a first storage section 83, a second storage section 84, a first channel calculating section 85, a second channel calculating section 86, a third channel calculating section 87, a determining section 88, and a minimum value detecting section 89, or the like.

The detected signal receiving section (measuring section) 81 receives the detected voltages from the reflected-light averaging circuit 561 and the transmitted-light averaging circuit 562, and takes samples of the detected voltages for each prescribed length interval of the traveling spun yarn 10 to convert into digital data (raw data). In the present embodiment, the sample is taken for each 1 mm interval of the traveling spun yarn 10.

Each time the detected signal receiving section 81 takes samples of the detected voltages, the light-receiving rate calculating section 82 calculates a light-receiving rate (%) of the reflected light based on the sampled raw data. The detected voltage of the reflected light from the spun yarn 10 having no foreign substance is defined as a standard voltage. The light-receiving rate is obtained by acquiring a change rate (%) that indicates how much the voltage value of the reflected light has changed from the standard voltage, and then dividing the acquired change rate by the yarn thickness (which is obtained based on the raw data of the transmitted light). When the yarn is thin, the reflected light decreases, and when the yarn is thick, the reflected light increases. Therefore, the change rate is divided by the yarn thickness in order to eliminate influence from the yarn thickness by correcting the change rate based on the yarn thickness, and to more accurately detect the change in the reflected light arising from influence from a foreign substance.

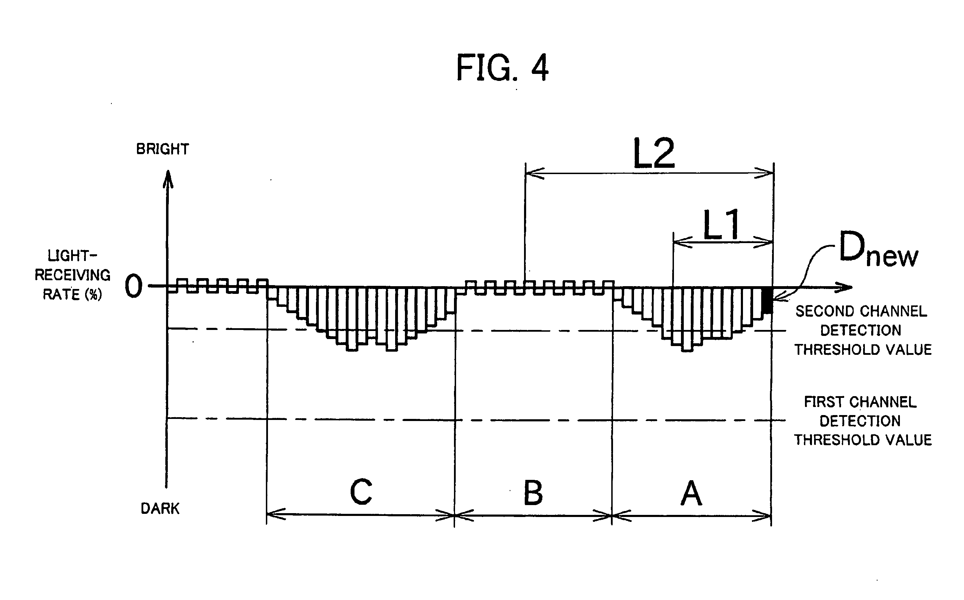

The first storage section 83 includes a memory area provided as a ring buffer that can store a plurality of pieces of data. The first storage section 83 stores light-receiving rate data of the reflected light calculated by the light-receiving rate calculating section 82 for a newest prescribed yarn length. Fig. 4 schematically illustrates the light-receiving rate data that is stored in the first storage section 83 when the spun yarn 10 illustrated in Fig. 7B is measured by the yarn clearer 52. Since Fig. 4 is a schematic diagram, actual data on the memory provided as the ring buffer in the first storage section 83 is not arranged as illustrated in Fig. 4.

A section A, a section B, and a section C of Fig. 4 respectively correspond to a section A, a section B, and a section C of Fig. 7B. Each bar graph of Fig. 4 represents each light-receiving rate data. As described above, in the present embodiment, since the sample is taken for each 1 mm of the traveling spun yarn, each bar graph represents a light-receiving rate of 1 mm of the spun yarn. A vertical axis of the graph represents the light-receiving rate, and the graph indicates that the lower the vertical axis is, the smaller the amount of the received light is (meaning "dark"). A horizontal axis of the graph represents time. The graph indicates that the closer the horizontal axis approaches towards right, the newer the data is, and that a rightmost "Dnew" represents the newest light-receiving rate data. In the following description, a "measurement position of the light-receiving rate data" refers to a position on the spun yarn 10 where the sample has been taken of the detected voltage that has been used to calculate the light-receiving rate data.

Next, a method of detecting the foreign substance performed by the analyzer 80 will be briefly described. The analyzer 80 of the yarn clearer 52 according to the present embodiment can distinguish and detect three types of foreign substance based on the detected voltages of the reflected light and the transmitted light received from the clearer head 521. Since a plurality of foreign substances can be distinguished and detected, detection mechanism for each foreign substance may be called "channel" in the following description.

A first channel detects a dark and short foreign substance. A second channel detects a colored yarn which is a slightly-dark and long foreign substance. A third channel detects a colored yarn that is linear at a middle portion as illustrated in Fig. 7B. By monitoring the detected voltages of the transmitted light and the reflected light, the analyzer 80 according to the present embodiment can detect various yarn defects (for example, a fly waste, a slub, or unevenness in yarn thickness) in addition to the foreign substances. However, the description of such detection will be omitted.

A basic concept of a method of detecting the foreign substance by the first and second channels is as follows. That is, an average value of the light-receiving rates in a range of a prescribed length of the spun yarn 10 is obtained, the average value and a threshold value are compared, and when the average value falls below the threshold value, a determination is made that the spun yarn includes a foreign substance. In other words, since the foreign substance has a certain degree of length and color, by obtaining the average amount of received light within the range of the prescribed length, a determination can be made as to whether or not the spun yarn includes a foreign substance having a certain color and length. Moreover, since the average value is obtained for a certain degree of range, even if abnormal light-receiving rate data influenced by a noise exists within the range, influence from such abnormal light-receiving rate data can be reduced. Accordingly, the above-described method of detecting the foreign substance is also advantageous in terms of noise reduction.

A specific description will be made with reference to Fig. 4. When detecting the foreign substance by the first channel, an average value is calculated for the light-receiving rate data measured within a section of a first channel length L1 located downstream of the spun yarn 10 from a measurement position of the newest light-receiving rate data Dnew. When the average value falls below a first channel detection threshold value, a determination is made that the spun yarn 10 includes a foreign substance. When detecting the foreign substance by the second channel, an average value is calculated for the light-receiving rate data measured within a section of a second channel length L2 located downstream of the spun yarn 10 from the measurement position of the newest light-receiving rate data Dnew. When the average value falls below a second channel detection threshold value, a determination is made that the spun yarn 10 includes a foreign substance. As illustrated in Fig. 4, the second channel length L2 is longer than the first channel length L1, and the first channel detection threshold value is lower than the second channel detection threshold value. Thus, the first channel can detect a dark and short foreign substance, and the second channel can detect a slightly-dark and long foreign substance (i.e., colored yarn).

The basic concept of the method of detecting the foreign substance by the first and second channels has been described above. In the present embodiment, the average value is not obtained, but a sum and a threshold value are compared. In other words, when obtaining the average value, a sum of the light-receiving rate data (a light-receiving rate sum) for the first and second channel lengths is calculated, and then, the light-receiving rate sum is divided by the number of pieces of data. By using the light-receiving rate sum instead of obtaining the average value, an amount of calculations is reduced, and processing load on the CPU can also be reduced. In the following description, the sum is used according to an embodiment of the present invention.

The first channel calculating section (received-light value detecting section) 85 extracts, from the light-receiving rate data stored in the first storage section 83, pieces of the light-receiving rate data that have been measured within a section which corresponds to the first channel length L1 and is located downstream of the spun yarn 10 from the measurement position of the newest light-receiving rate data Dnew. Then, the first channel calculating section 85 calculates a light-receiving rate sum (received-light value) of the section. In the present embodiment, the first channel length L1 is set to be 10 mm of the spun yarn 10. Accordingly, the first channel calculating section 85 extracts ten pieces of newest light-receiving rate data from the first storage section 83 and calculates the sum. The light-receiving rate sum is calculated each time new light-receiving rate data is added to the first storage section 83.

The second channel calculating section 86 extracts, from the light-receiving rate data stored in the first storage section 83, pieces of the light-receiving rate data that have been measured within a section which corresponds to the second channel length L2 and is located downstream of the spun yarn 10 from the measurement position of the newest light-receiving rate data Dnew. Then, the second channel calculating section 86 calculates a light-receiving rate sum of the section. In the present embodiment, the second channel length L2 is set to be 25 mm of the spun yarn 10. Accordingly, the second channel calculating section 86 extracts 25 pieces of newest light-receiving rate data from the first storage section 83 and calculates the sum. The light-receiving rate sum is also calculated each time new light-receiving rate data is added to the first storage section 83.

The determining section 88 compares the light-receiving rate sums, which have been calculated by the first channel calculating section 85 and the second channel calculating section 86, with the threshold value to determine whether or not the spun yarn 10 includes a foreign substance. More specifically, the light-receiving rate sum of the ten pieces of the light-receiving rate data calculated by the first channel calculating section 85 is compared with the threshold value (which is ten times the above-described first channel determination threshold value). When the sum falls below the threshold value, the determining section 88 determines that the spun yarn 10 includes a dark and short foreign substance. Similarly, the light-receiving rate sum of the 25 pieces of light-receiving rate data calculated by the second channel calculating section 86 is compared with the threshold value (which is 25 times the above-described second channel determination threshold value). When the sum falls below the threshold value, the determining section 88 determines that the spun yarn 10 includes a slightly-dark and long foreign substance (colored yarn).

When the colored yarn has a linear portion (section B) as illustrated in Fig. 4, the first and second channels cannot detect such colored yarn as a foreign substance. In other words, because the section A, in which the colored yarn is in spirals, is not dark enough to be detected by the first channel detection threshold value, the section A cannot be detected as a colored yarn by the first channel. Moreover, because a portion of the section B (which has a great light-receiving rate) is located inside the range of the second channel length L2, the sum calculated by the second channel calculating section becomes great and exceeds the second channel detection threshold value. Therefore, such colored yarn cannot be detected also by the second channel.

In the yarn clearer 52 according to the present embodiment, in order to detect the colored yarn that is linear at the middle portion, the third channel obtains a sum excluding the light-receiving rate data measured in the prescribed range, and compares the sum and the prescribed threshold value. In other words, the sum of the light-receiving rate data is obtained by skipping the middle portion (section B) in which the colored yarn is linear, and the determination of the foreign substance can be performed by ignoring the linear portion.

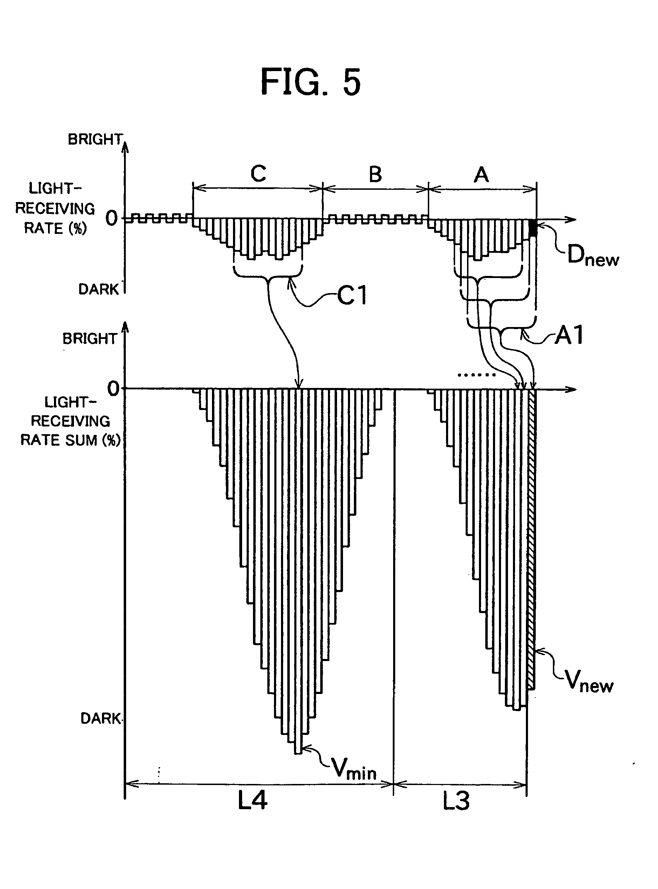

A description will be made with reference to Fig. 5. Similarly to Fig. 4, an upper graph of Fig. 5 schematically illustrates the data of the amount of the received light stored in the first storage section 83. For example, a sum is calculated for the light-receiving rate data measured for a prescribed range A1 located downstream from the measurement position of the newest light-receiving rate data Dnew and the light-receiving rate data measured for a prescribed range C1 located downstream from the measurement position of the newest light-receiving rate data Dnew with a prescribed interval spaced therebetween. Then, the calculated sum and the threshold value are compared. The light-receiving rate data measured in the range A1 corresponds to the detection result in the section A in which the colored yarn is in spirals. The light-receiving rate data measured in the range C1 corresponds to the detection result in the section C in which the colored yarn is in spirals. By obtaining the sum of the light-receiving rate data measured in the range A1 and the light-receiving rate data measured in the range C1, the sum can be calculated while excluding the light-receiving rate data measured in the section B. Therefore, by comparing the sum and an appropriate threshold value, even the colored yarn having a linear middle portion can be accurately detected.

However, in actual spun yarns, a length of the section (section B) in which the colored yarn is linear varies. Accordingly, the colored yarn cannot be accurately detected in a configuration in which the range C1 is set in advance. Therefore, in the present embodiment, the second storage section 84, the third channel calculating section 87, and the minimum value detecting section 89 have the following configuration.

The second storage section (storage section) 84 includes a memory area provided as a ring buffer that can store a plurality of pieces of data. The second storage section 84 stores the light-receiving rate sum of ten pieces of the light-receiving rate data (the third length) calculated by the first channel calculating section 85 for a newest prescribed yarn length. Each time the first channel calculating section 85 calculates the light-receiving rate sum of ten pieces of the light-receiving rate data, the second storage section 84 adds the calculated light-receiving rate sum to the ring buffer. As described above, since the value calculated by the first channel calculating section 85 is stored and used, the light-receiving rate sum does not need to be newly calculated, which thereby reduces processing load.

The lower graph in Fig. 5 is a graph schematically representing the light-receiving rate sum stored in the second storage section 84. Each bar graph in the lower graph of Fig. 5 represents each light-receiving rate sum. As described above, since the light-receiving rate sum is calculated for each 1 mm of the travelling spun yarn 10, each bar graph represents the light-receiving rate sum calculated for each 1 mm of the spun yarn 10. A vertical axis of the graph represents the light-receiving rate sum, and the graph indicates that the lower the vertical axis is than a light-receiving rate 0%, which is to be a standard, the smaller the received light amount is (meaning "dark"). A horizontal axis of the graph represents time. The graph indicates that the closer the horizontal axis approaches towards right, the newer the data is, and that a rightmost "Vnew" represents the newest light-receiving rate sum. In the description, a "measurement position of the light-receiving rate sum" indicates that the light-receiving rate sum has been measured at the measurement position of the newest light-receiving rate data among the light-receiving rate data measured in the light-receiving rate sum.

The third channel calculating section (calculating section) 87 calculates a sum of a newest light-receiving rate sum Vnew and a minimum light-receiving rate sum Vmin (which is obtained by the minimum value detecting section 89 to be described later) obtained for a range of a determining length (second length) L4.

The range of the determining length L4 in the spun yarn 10 is set downstream from a measurement position of the newest light-receiving rate sum Vnew with an interval of a set length (first length) L3 spaced apart therebetween. In the third channel, the light-receiving rate sums of the range of the set length L3 from the measurement position of the newest light-receiving rate sum Vnew are not considered during the foreign substance detecting process. In other words, the light-receiving rate sums of the range of the set length L3 are ignored in order to skip the linear portion (section B) of the colored yarn. A proper value is set as the set length L3 in a manner that an interval between the newest light-receiving rate sum Vnew and the minimum light-receiving rate sum Vmin within the range of the determining length L4 will be at least one piece of light-receiving rate data or greater (i.e., in the present embodiment, 1 mm or greater). In the present embodiment, the set length L3 of the spun yarn 10 is a range of 20 mm (a range corresponding to 20 light-receiving rate sums) located downstream from the measurement position of the newest light-receiving rate sum Vnew of the spun yarn 10.

As described above, a length of the linear portion (section B) of the colored yarn cannot be confirmed in advance. Accordingly, in the present embodiment, the section C of the colored yarn is detected while providing some margin by the range of the determining length L4. The determining length L4 according to the present embodiment refers to a range of 40 mm of the spun yarn 10 (i.e., a range of 40 light-receiving rate sums). For example, a sum of each of the light-receiving rate sums measured in the range of the determining length L4 and the newest light-receiving rate sum Vnew may be calculated, and when even one of the sums falls below the prescribed threshold value, a determination is made that the spun yarn 10 includes a foreign substance. However, when the above comparing and determining process is performed each time with respect to each light-receiving rate sum of the range of the determining length L4, calculation load becomes excessive. Therefore, in the present embodiment, only the sum of the minimum light-receiving rate sum Vmin within the range of the determining length L4 and the newest light-receiving rate sum Vnew is calculated.

The minimum value detecting section 89 obtains the minimum light-receiving rate sum Vmin in the range of the determining length L4. If the minimum value in the range of the determining length L4 is obtained each time the foreign substance determining process is performed, extra calculation load is placed. Therefore, the minimum value detecting section 89 is configured as follows.

The minimum value detecting section 89 stores the most-recently obtained minimum light-receiving rate sum Vmin. The third channel calculating section 87 retrieves the light-receiving rate sum Vmin stored in the minimum value detecting section 89 to use in the calculation of the sum. When the measurement position of the stored minimum light-receiving rate sum Vmin falls outside the range of the determining length L4, the minimum value detecting section 89 re-calculates a minimum light-receiving rate sum within the range of the determining length L4, and updates the stored minimum light-receiving rate sum Vmin. When a light-receiving rate sum measured at a measurement position newly entering the range of the determining length L4 is smaller than the stored minimum light-receiving rate sum Vmin, the minimum light-receiving rate sum Vmin is updated.

The determining section 88 compares the sum of the newest light-receiving rate sum Vnew and the minimum light-receiving rate sum Vmin calculated by the third channel calculating section 87 with the prescribed threshold value, and when the sum falls below the threshold value, the determining section 88 determines that the spun yarn 10 includes a foreign substance (colored yarn).

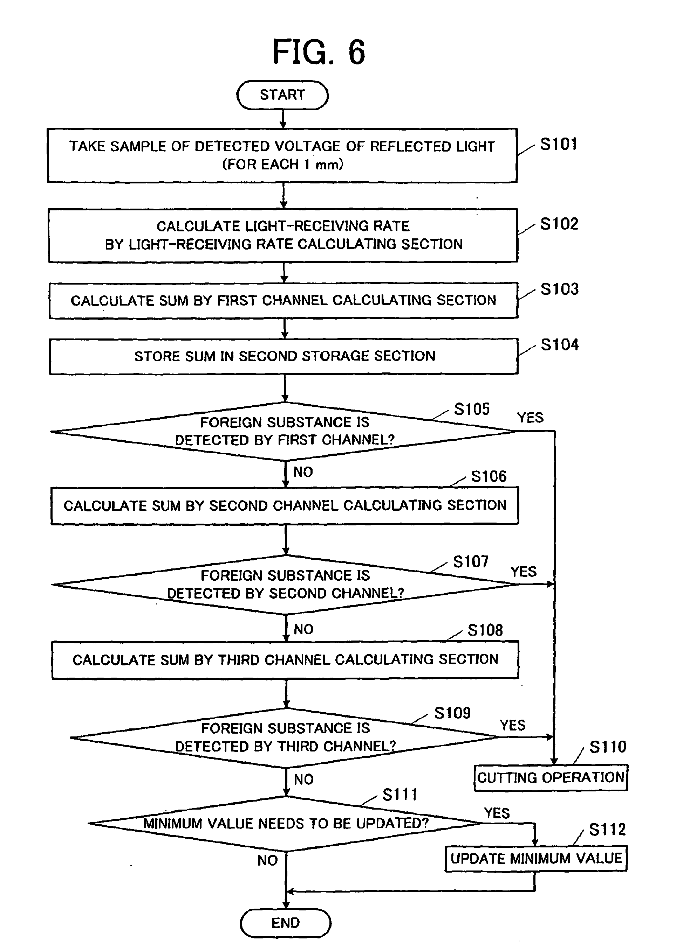

With reference to a flowchart of Fig. 6, a method of detecting the foreign substance by the yarn clearer 52 will be described.

The detected signal receiving section 81 takes sample of the detected voltages of the reflected light and the transmitted light received from the traveling spun yarn (a measuring step, step S101). When the sample is taken, the light-receiving rate calculating section 82 calculates the light-receiving rate of the reflected light in accordance with the sampled raw data (step S102). At this time, the newest light-receiving rate data Dnew is added to the first storage section 83.

Then, the first channel calculating section 85 calculates the sum (received-light value) of the light-receiving rate data measured within the range of the first channel length L1 located downstream from the measurement position of the newest light-receiving rate data Dnew (a received-light value detecting step, step S103).

When the newest light-receiving rate sum Dnew is calculated by the first channel calculating section 85, the calculated sum is added to the second storage section 84 (a storing step, step S104).

The determining section 88 compares the light-receiving rate sum calculated by the first channel calculating section 85 with the prescribed threshold value (step S105), and when the sum falls below the threshold value, the determining section 88 determines that the spun yarn 10 includes a foreign substance. When the foreign substance is detected, the yarn defect detection signal is transmitted from the yarn clearer 52 to the unit control section 73, and an operation of cutting off the portion of the foreign substance and a yarn splicing operation are performed (step S110).

Then, the second channel calculating section 86 calculates the sum of the light-receiving rate data measured in the range of the second channel length L2 located downstream from the measurement position of the newest light-receiving rate data Dnew (step S106). The determining section 88 compares the light-receiving rate sum calculated by the second channel calculating section 86 with the prescribed threshold value (S107), and when the sum falls below the threshold value, the determining section 88 determines that the spun yarn 10 includes a foreign substance. When the foreign substance is detected, the yarn defect detection signal is transmitted from the yarn clearer 52 to the unit control section 73, and the operation of cutting off the portion of the foreign substance and the yarn splicing operation are performed (step S110).

When the first and second channels could not detect any foreign substance, the third channel calculating section 87 calculates the sum. The third channel calculating section 87 retrieves the newest light-receiving rate sum from the second storage section 84, obtains the minimum light-receiving rate sum stored in the minimum value detecting section 89, and calculates the sum of the newest light-receiving rate sum and the minimum light-receiving rate sum (a calculating step, step S108).

The determining section 88 compares the light-receiving rate sum calculated by the third channel calculating section 87 with the prescribed threshold value (a comparing step, step S109), and when the sum falls below the prescribed threshold value, the determining section 88 determines that the spun yarn 10 includes a foreign substance. When the foreign substance is detected, the yarn defect detection signal is transmitted from the yarn clearer 52 to the unit control section 73, and the operation of cutting off the portion of the foreign substance and the yarn splicing operation are performed (step S110).

Then, the minimum value detecting section 89 determines whether or not the stored minimum light-receiving rate sum needs to be updated (step S111). As described above, when the stored minimum light-receiving rate sum falls outside the range of the determining length L4, or when a light-receiving rate sum that is smaller than the stored minimum light-receiving rate sum enters into the range of the determining length L4, the stored minimum light-receiving rate sum is updated (step S112).

By executing the above flow for each 1 mm of the traveling spun yarn 10, the spun yarn 10 can be continuously monitored, and the foreign substance can be detected.

As described above, the yarn clearer 52 according to the present embodiment includes the light emitting sections 531, 532, the light receiving sections 541, 542, the detected signal receiving section 81, the first channel calculating section 85, the second storage section 84, the third channel calculating section 87, and the determining section 88. The light emitting sections 531, 532 emit light to the traveling spun yarn 10. The light receiving sections 541, 542 receive a reflection of the light that is emitted from the light emitting sections 531, 532 and reflected by the spun yarn 10. The detected signal receiving section 81 takes the sample of the amount of the light received by the light receiving sections 541, 542 for each 1 mm of the spun yarn 10. The first channel calculating section 85 calculates the light-receiving rate sum in accordance with the amount of the received light sampled by the detected signal receiving section 81. The second storage section 84 stores a plurality of light-receiving rate sums calculated by the first channel calculating section 85. The third channel calculating section 87 calculates the sum of the newest light-receiving rate sum calculated by the first channel calculating section 85 and the light-receiving rate sum that is stored in the second storage section 84 after being measured at an interval of at least 20 mm (the setting length L3), which is longer than a sampling interval (1 mm), spaced apart from the measurement position of the newest light-receiving rate sum. The determining section 88 determines the presence or the absence of the foreign substance in accordance with the sum calculated by the third channel calculating section 87.

In other words, the light-receiving rate sum is obtained with an interval of the setting length L3 spaced apart, and the presence or the absence of the foreign substance is determined in accordance with the sum. Therefore, the foreign substance determination can be performed by skipping the portion of the setting length L3. Accordingly, even the colored yarn that is linear at the middle portion can be detected as the foreign substance without being influenced from the light-receiving rate data measured at the middle portion. Thus, the colored yarn can be detected more accurately.

The yarn clearer 52 according to the present embodiment further includes the minimum value detecting section 89. The minimum value detecting section 89 obtains the minimum light-receiving rate sum from among the light-receiving rate sums that are stored in the second storage section 84 after being measured within the range of the determining length L4 located downstream from the measurement position of the newest light-receiving rate sum with at least an interval of 20 mm (the setting length L3), which is longer than the sampling interval (1 mm), spaced apart therebetween. The third channel calculating section 87 calculates the sum of the newest light-receiving rate sum and the minimum light-receiving rate sum.

In other words, by providing the interval of the setting length L3 between the newest light-receiving rate sum and the minimum light-receiving rate sum, the sum that has skipped the linear portion of the colored yarn can be obtained. Moreover, since the past light-receiving rate sum within the range of the determining length L4 having some margin is considered, the colored yarn can be detected while taking into account a fact that the lengths of the middle portion in which the colored yarn is linear varies for each colored yarn. Further, since the sum of the minimum value of the past light-receiving rate sum and the newest light-receiving rate sum is calculated, it is not necessary to obtain the sum of the newest light-receiving rate sum and each of the past light-receiving rate sums of the range of the determining length L4 in order to determine the presence or the absence of the foreign substance. Therefore, calculation efficiency can be improved.

The minimum value detecting section 89 stores the obtained minimum light-receiving rate sum. When the measurement position of the minimum light-receiving rate sum falls outside the range of the determining length L4, the minimum value detecting section 89 newly obtains a minimum light-receiving rate sum in the range of the determining length L4, and updates the stored minimum light-receiving rate sum. Accordingly, since it is not necessary to obtain the minimum value by comparing each time all of the light-receiving rate sums which are measured at the measurement positions within the range of the determining length L4, the calculation efficiency can be further improved.

When a light-receiving rate sum measured at a measurement position newly entering into the range of the determining length L4 is smaller than the stored minimum light-receiving rate sum, the minimum value detecting section 89 updates the stored minimum light-receiving rate sum. Accordingly, since the stored minimum light-receiving rate sum can be easily updated, the calculation efficiency can be further improved.

The first channel calculating section 85 calculates the light-receiving rate sum by summing up the light-receiving rate data measured within the range located downstream for the first channel length L1 from the measurement position of the light-receiving rate sum. Since the light-receiving rate sum is calculated by considering the light-receiving rate data measured in the range having a certain degree, influence from noise can be reduced, and detection accuracy can be improved.

The determining section 88 compares the sum calculated by the third channel calculating section 87 with the prescribed threshold value, and determines the presence or the absence of the foreign substance. Accordingly, the foreign substance can be detected by a simple process of comparing the sum and the threshold value.

The spinning machine 1 according to the present embodiment includes the above-described yarn clearer 52. Accordingly, since the spinning machine 1 can detect the colored yarn that is linear at the middle portion, a package quality can be improved.

A foreign substance detecting method of the present invention includes a measuring step, a received-light value detecting step, a storing step, a calculating step, and a comparing step. In the measuring step, light is emitted to a traveling yarn 10, the light that has been reflected by the spun yarn 10 is received, and a sample of an amount of the received light is taken for each 1 mm of the spun yarn 10. In the received-light value detecting step, a light-receiving rate sum is calculated in accordance with a measured value of the amount of the received light. In the storing step, the light-receiving rate sum is stored in the second storage section 84. In the calculating step, a sum is obtained for a newest light-receiving rate sum and a light-receiving rate sum which has been stored in the second storage section 84 after being measured at a position located downstream from the measurement position of the newest light-receiving rate sum with an interval of at least 20 mm (the setting length L3), which is longer than the sampling interval (1 mm), spaced apart therebeween. In the comparing step, the sum and the prescribed threshold value are compared in order to detect a foreign substance.

Since the above method obtains the sum of the light-receiving rate sums at prescribed intervals, and determines the presence or the absence of the foreign substance in accordance with the sum, the foreign substance determination can be performed by skipping the portion of the setting length L3. Thus, even the colored yarn that is linear at the middle portion can be detected as the foreign substance without being influenced by the light-receiving rate data of the middle portion. Accordingly, the colored yarn can be detected more accurately.

The preferred embodiments of the present invention have been described above, however, the above-described configuration can be changed, for example, as follows.

The sampling interval, the first channel length L1, the second channel length L2, the setting length L3, and the determining length L4 or the like are not limited to the values described above, and can be changed as appropriate. An operator may input a desired value to the machine control device 60 to appropriately change the above values.

In the above-described embodiment, the third channel uses the light-receiving rate sum calculated by the first channel calculating section 85. However, the third channel may calculate a sum of the light-receiving rate data measured in a prescribed range independently from the first channel (i.e., the length of the range A1 and the length of the range C1 do not need to match the first channel length L1). However, by using the already calculated light-receiving rate sum, an amount of calculations can be reduced. Accordingly, it is preferable to use the light-receiving rate sum already calculated by the first channel calculating section 85.

In the above-described embodiment, the third channel calculating section 87 calculates the sum of the light-receiving rate sums. However, the present invention is not limited to the above-described embodiment. For example, the third channel calculating section 87 may calculate a sum of the newest light-receiving rate data Dnew and the light-receiving rate data measured at the position located downstream from the newest light-receiving rate data Dnew with a prescribed interval spaced apart therebetween. In such a case, however, if abnormal light-receiving rate data arising from the influence from noise or the like is included, the colored yarn cannot be detected accurately. Therefore, it is preferable to obtain the sum of the light-receiving rate sums of the light-receiving rate data measured in the range (in the present embodiment, in the first channel length L1) having a certain degree as described in the above embodiment in order to use the obtained sum in the foreign substance determination.

In the above embodiment, various efforts are made in order to reduce the calculation load, however, such efforts are not essential, and may be omitted if the CPU or the like has a sufficient processing ability. For example, the process of detecting the minimum value by the minimum value detecting section 89 may be omitted. Instead, the sums of the newest light-receiving rate sum and each of the light-receiving rate sums measured in the range of the determining length L4 may be calculated, and each of the sums may be compared with the threshold value. Alternatively, for example, the first channel calculating section 85 may calculate an average value of the light-receiving rate data measured in the range of the first channel length L1. In such a case, moving average values of the light-receiving rate data are sequentially stored in the second storage section 84. The third channel can detect the colored yarn that is linear at the middle portion with a substantially similar accuracy to that of the above embodiment by using the moving average value of this modified example.

The number of the light emitting sections provided in the clearer head 521 is not limited to two, and one light emitting section or three or more light emitting sections may be provided. However, in the case of the clearer head 521 includes only one light emitting section, a light-radiated portion is small, and a foreign substance in a portion opposite to the light-radiated portion cannot be detected. As a result, the accuracy of the foreign substance detection is reduced. Accordingly, it is preferable to provide at least two light emitting sections.

In the above-described embodiment, the clearer head 521 and the analyzer 80 are individually provided, however, the clearer head 521 may include the analyzer 80.

The present invention is not limited to spinning machines, but may be applied to other textile machines such as an automatic winder.

## Claims

1. A foreign substance detecting device **characterized by** comprising:
a light emitting section (531, 532) configured to emit light to a traveling yarn;
a light receiving section (541, 542) configured to receive light which is a reflection of the light emitted from the light emitting section (531, 532) and reflected by the yarn;
a measuring section (81) configured to obtain a measured value by measuring an amount of the light received by the light receiving section (541, 542) for each prescribed yarn length interval;
a received-light value detecting section (85) configured to obtain a received-light value in accordance with the measured value measured by the measuring section (81);
a storage section (84) configured to store a plurality of received-light values obtained by the received-light value detecting section (85);
a calculating section (87) configured to calculate a sum of received-light values; and
a determining section (88) configured to determine a presence or an absence of a foreign substance in accordance with the sum calculated by the calculating section (87),
**characterized in that**
the calculating section (87) is configured to calculate a sum of a newest received-light value, which has been obtained by the received-light value detecting section (85), and a received-light value, which is stored in the storage section (84) after being calculated in accordance with the measured value measured at a position located downstream for a prescribed length than the yarn length interval from the measurement position of the measured value corresponding to the newest received-light value.

2. The foreign substance detecting device according to claim 1, **characterized by** further comprising a minimum value detecting section (89) configured to obtain a minimum received-light value from the plurality of the received-light values, which have been obtained in accordance with measured values measured within a range of a prescribed second length located downstream for a prescribed first length than the yarn length interval from the measurement position of the measured value corresponding to the newest received-light value and stored in the storage section (84),
**characterized in that** the calculating section (87) is configured to calculate a sum of the newest received-light value and the minimum received-light value.

3. The foreign substance detecting device according to claim 2, **characterized in that** the minimum value detecting section (89) is configured to store the obtained minimum received-light value, and when the measurement position of the measured value corresponding to the minimum received-light value falls outside the range of the second length, the minimum value detecting section (89) is configured to newly obtain a minimum received-light value in the range of the second length and updates the minimum received-light value stored in the storage section (84).

4. The foreign substance detecting device according to claim 2 or claim 3, **characterized in that** when a received-light value, which has been obtained in accordance with a measured value measured at a measurement position newly entering into the range of the second length, is smaller than the minimum received-light value stored in the storage section (84), the minimum value detecting section (89) is configured to update the minimum received-light value stored in the storage section (84).

5. The foreign substance detecting device according to any one of claim 1 through claim 4, **characterized in that** the received-light value detecting section (85) is configured to obtain the received-light value by considering a measured value measured in a range located downstream for a prescribed third length from the measurement position of the measured value corresponding to the received-light amount.

6. The foreign substance detecting device according to any one of claim 1 through claim 5, **characterized in that** the determining section (88) is configured to determine the presence or the absence of the foreign substance by comparing the sum calculated by the calculating section (87) and a prescribed threshold value.

7. A textile machine comprising the foreign substance detecting device (52) according to any one of claim 1 through claim 6.

8. A foreign substance detecting method **characterized by** comprising:
a measuring step of emitting light to a traveling yarn, receiving light reflected from the traveling yarn, and measuring an amount of received light for each prescribed yarn length interval;
a received-light value detecting step of obtaining a received-light value in accordance with a measured value of the amount of the received light;
a storing step of storing the received-light value in a storage section;
a calculating step of calculating a sum of received-light values; and
a comparing step of comparing the sum and a prescribed threshold value,
**characterized in that**
**that** calculating step comprises calculating a sum of a newest received-light value and a received-light value stored in the storage section after being obtained in accordance with a measured value measured at a position located downstream for a prescribed length than the yarn length interval from a measurement position of a measured value corresponding to the newest received-light value.

## Patentansprüche

1. Eine Fremdsubstanzerfassungsvorrichtung, die **dadurch gekennzeichnet ist, dass** sie Folgendes umfasst:
einen Lichtemissionsabschnitt (531, 532), der dazu konfiguriert ist, Licht an ein laufendes Garn zu emittieren;
einen Lichtempfangsabschnitt (541, 542), der dazu konfiguriert ist, Licht zu empfangen, das eine Reflexion des aus dem Lichtemissionsabschnitt (531, 532) emittierten und durch das Garn reflektierten Lichts ist;
einen Messabschnitt (81), der dazu konfiguriert ist, einen Messwert zu erhalten, indem er eine Menge des Lichts, das seitens des Lichtempfangsabschnitts (541, 542) empfangen wird, für jedes vorgeschriebenes Garnlängenintervall misst;
ein Empfangslichtwerterfassungsabschnitt (85), der dazu konfiguriert ist, einen Empfangslichtwert gemäß dem Messwert, der seitens des Messabschnitts (81) gemessen wird, zu erhalten;
einen Speicherabschnitt (84), der dazu konfiguriert ist, eine Mehrzahl von Empfangslichtwerten, die seitens des Empfangslichtwerterfassungsabschnitts (85) erhalten werden, zu speichern;
einen Berechnungsabschnitt (87), der dazu konfiguriert ist, eine Summe von Empfangslichtwerten zu berechnen; und
einen Bestimmungsabschnitt (88), der dazu konfiguriert ist, ein Vorliegen oder ein Fehlen einer Fremdsubstanz gemäß der seitens des Berechnungsabschnitts (87) berechneten Summe zu bestimmen,
**dadurch gekennzeichnet, dass**
der Berechnungsabschnitt (87) dazu konfiguriert ist, eine Summe eines neuesten Empfangslichtwerts zu berechnen, der seitens des Empfangslichtwerterfassungsabschnitts (85) erhalten wurde, und einen Empfangslichtwert, der in dem Speicherabschnitt (84) gespeichert wird, nachdem er gemäß dem Messwert berechnet wurde, der an einer Position gemessen wird, die um eine über das Garnlängenintervall hinausgehende vorgeschriebene Länge flussabwärts von der Messposition des Messwerts, der dem neuesten Empfangslichtwert entspricht, angeordnet ist.

2. Die Fremdsubstanzerfassungsvorrichtung gemäß Anspruch 1, **dadurch gekennzeichnet, dass** sie ferner einen Mindestwerterfassungsabschnitt (89) umfasst, der dazu konfiguriert ist, einen Mindestempfangslichtwert von der Mehrzahl der Empfangslichtwerte zu erhalten, die gemäß Messwerten erhalten werden, die in einem Bereich einer vorgeschriebenen zweiten Länge gemessen werden, die um eine über das Garnlängenintervall hinausgehende vorgeschriebene erste Länge flussabwärts von der Messposition des Messwerts, der dem neuesten Empfangslichtwert entspricht und in dem Speicherabschnitt (84) gespeichert ist, angeordnet ist,
**dadurch gekennzeichnet, dass** der Berechnungsabschnitt (87) dazu konfiguriert ist, eine Summe des neuesten Empfangslichtwerts und des Mindestempfangslichtwerts zu berechnen.

3. Die Fremdsubstanzerfassungsvorrichtung gemäß Anspruch 2, **dadurch gekennzeichnet, dass** der Mindestwerterfassungsabschnitt (89) dazu konfiguriert ist, den erhaltenen Mindestempfangslichtwert zu speichern, und wenn die Messposition des Messwerts, der dem Mindestempfangslichtwert entspricht, außerhalb des Bereichs der zweiten Länge liegt, ist der Mindestwerterfassungsabschnitt (89) dazu konfiguriert, einen Mindestempfangslichtwert in dem Bereich der zweiten Länge neu zu erhalten, und er aktualisiert den in dem Speicherabschnitt (84) gespeicherten Mindestempfangslichtwert.

4. Die Fremdsubstanzerfassungsvorrichtung gemäß Anspruch 2 oder 3, **dadurch gekennzeichnet, dass**, wenn ein Empfangslichtwert, der gemäß einem Messwert erhalten wird, der an einer Messposition gemessen wird, die neu in den Bereich der zweiten Länge eintritt, kleiner ist als der in dem Speicherabschnitt (84) gespeicherte Mindestempfangslichtwert, der Mindestwertempfangsabschnitt (89) dazu konfiguriert ist, den in dem Speicherabschnitt (84) gespeicherten Mindestempfangslichtwert zu aktualisieren.

5. Die Fremdsubstanzerfassungsvorrichtung gemäß einem der Ansprüche 1 bis 4, **dadurch gekennzeichnet, dass** der Empfangslichtwerterfassungsabschnitt (85) dazu konfiguriert ist, den Empfangslichtwert zu erhalten, indem er einen Messwert berücksichtigt, der in einem Bereich gemessen wird, der um eine vorgeschriebene dritte Länge flussabwärts von der Messposition des Messwerts, der der Empfangslichtmenge entspricht, angeordnet ist.

6. Die Fremdsubstanzerfassungsvorrichtung gemäß einem der Ansprüche 1 bis 5, **dadurch gekennzeichnet, dass** der Bestimmungsabschnitt (88) dazu konfiguriert ist, das Vorliegen oder das Fehlen der Fremdsubstanz zu bestimmen, indem er die seitens des Berechnungsabschnitts (87) berechnete Summe und einen vorgeschriebenen Schwellwert vergleicht.

7. Eine Textilmaschine, die die Fremdsubstanzerfassungsvorrichtung (52) gemäß einem der Ansprüche 1 bis 6 aufweist.

8. Ein Fremdsubstanzerfassungsverfahren, das **dadurch gekennzeichnet ist, dass** es folgende Schritte aufweist:
einen Messschritt des Emittierens von Licht an ein laufendes Garn, des Empfangens von Licht, das von dem laufenden Garn reflektiert wird, und des Messens einer Menge an empfangenem Licht für jedes vorgeschriebene Garnlängenintervall;
einen Empfangslichtwerterfassungsschritt des Erhaltens eines Empfangslichtwerts gemäß einem Messwert der Menge des empfangenen Lichts;
einen Speicherschritt des Speicherns des Empfangslichtwerts in einem Speicherabschnitt;
einen Berechnungsschritt des Berechnens einer Summe von Empfangslichtwerten;
und
einen Vergleichsschritt des Vergleichens der Summe und eines vorgeschriebenen Schwellwerts,
**dadurch gekennzeichnet, dass**
dieser Berechnungsschritt ein Berechnen einer Summe eines neuesten Empfangslichtwerts und eines Empfangslichtwerts umfasst, der in dem Speicherabschnitt gespeichert wird, nachdem er gemäß einem Messwert erhalten wurde, der an einer Position gemessen wird, die um eine über das Garnlängenintervall hinausgehende vorgeschriebene Länge flussabwärts von einer Messposition eines Messwerts, der dem neuesten Empfangslichtwert entspricht, angeordnet ist.

## Revendications

1. Dispositif de détection de substances étrangères, **caractérisé par le fait qu'**il comprend:
un segment émetteur de lumière (531, 532) configuré pour émettre de la lumière vers un fil se déplaçant;
un segment récepteur de lumière (541, 542) configuré pour recevoir de la lumière qui est une réflexion de la lumière émise par le segment émetteur de lumière (531, 532) et réfléchie par le fil;
un segment de mesure (81) configuré pour obtenir une valeur mesurée en mesurant une quantité de la lumière reçue par le segment récepteur de lumière (541, 542) pour chaque intervalle de longueur de fil prescrit;
un segment de détection de valeur de lumière reçue (85) configuré pour obtenir une valeur de lumière reçue conformément à la valeur mesurée par le segment de mesure (81);
un segment de mémoire (84) configuré pour mémoriser une pluralité de valeurs de lumière reçue obtenues par le segment de détection de valeur de lumière reçue (85);
un segment de calcul (87) configuré pour calculer une somme de valeurs de lumière reçue; et
un segment de détermination (88) configuré pour déterminer une présence ou une absence d'une substance étrangère conformément à la somme calculée par le segment de calcul (87),
**caractérisé par le fait que**
le segment de calcul (87) est configuré pour calculer une somme de la valeur de lumière reçue la plus récente qui a été obtenue par le segment de détection de valeur de lumière reçue (85), et une valeur de lumière reçue qui est mémorisée dans le segment de mémoire (84) après avoir été calculée conformément à la valeur mesurée à une position située en aval pour une longueur prescrite de l'intervalle de longueur de fil à partir de la position de mesure de la valeur mesurée correspondant à la valeur de lumière reçue la plus récente.

2. Dispositif de détection de substances étrangères selon la revendication 1, **caractérisé par le fait qu'**il comprend par ailleurs un segment de détection de valeur minimale (89) configuré pour obtenir une valeur minimale de lumière reçue à partir de la pluralité de valeurs de lumière reçue qui ont été obtenues conformément aux valeurs mesurées dans une plage d'une deuxième longueur prescrite située en aval pour une première longueur prescrite de l'intervalle de longueur de fil à partir de la position de mesure de la valeur mesurée correspondant à la valeur de lumière reçue la plus récente et mémorisée dans le segment de mémoire (84),
**caractérisé par le fait que** le segment de calcul (87) est configuré pour calculer une somme de la valeur de lumière reçue la plus récente et de la valeur minimale de lumière reçue.

3. Dispositif de détection de substances étrangères selon la revendication 2, **caractérisé par le fait que** le segment de détection de valeur minimale (89) est configuré pour mémoriser la valeur minimale de lumière reçue obtenue et, lorsque la position de mesure de la valeur mesurée correspondant à la valeur minimale de lumière reçue se situe en dehors de la plage de la deuxième longueur, le segment de détection de valeur minimale (89) est configuré pour obtenir à nouveau une valeur minimale de lumière reçue dans la plage de la deuxième longueur et pour mettre à jour la valeur minimale de lumière reçue mémorisée dans le segment de mémoire (84).

4. Dispositif de détection de substances étrangères selon la revendication 2 ou la revendication 3, **caractérisé par le fait que**, lorsqu'une valeur de lumière reçue qui a été obtenue conformément à une valeur mesurée à une position de mesure nouvellement entrée dans la plage de la deuxième longueur est inférieure à la valeur minimale de lumière reçue mémorisée dans la segment de mémoire (84), le segment de détection de valeur minimale (89) est configuré pour mettre à jour la valeur minimale de lumière reçue mémorisée dans le segment de mémoire (84).

5. Dispositif de détection de substances étrangères selon l'une quelconque des revendications 1 à 4, **caractérisé par le fait que** le segment de détection de valeur de lumière reçue (85) est configuré pour obtenir la valeur de lumière reçue en considérant une valeur mesurée dans une plage située en aval pour une troisième longueur prescrite à partir de la position de mesure de la valeur mesurée correspondant à la quantité de lumière reçue.

6. Dispositif de détection de substances étrangères selon l'une quelconque des revendications 1 à 5, **caractérisé par le fait que** le segment de détermination (88) est configuré pour déterminer la présence ou l'absence de la substance étrangère en comparant la somme calculée par le segment de calcul (87) et une valeur limite prescrite.

7. Machine textile comprenant le dispositif de détection de substances étrangères (52) selon l'une quelconque des revendications 1 à 6.

8. Procédé de détection de substances étrangères **caractérisé par le fait qu'**il comprend:
une étape de mesure d'émission de lumière vers un fil se déplaçant recevant de la lumière réfléchie du fil se déplaçant, et de mesure d'une quantité de lumière reçue pour chaque intervalle de longueur de fil prescrit;
une étape de détection de valeur de lumière reçue consistant à obtenir une valeur de lumière reçue conformément à une valeur mesurée de la quantité de la lumière reçue;
une étape de mémorisation de la valeur de lumière reçue dans un segment de mémoire;
une étape de calcul d'une somme de valeurs de lumière reçue; et
une étape de comparaison de la somme et d'une valeur limite prescrite,
**caractérisé par le fait que**
l'étape de calcul comprend le calcul d'une somme de la valeur de lumière reçue la plus récente et d'une valeur de lumière reçue mémorisée dans le segment de mémoire après avoir été obtenue conformément à une valeur mesurée à une position située en aval pour une longueur prescrite de l'intervalle de longueur de fil à partir d'une position de mesure d'une valeur mesurée correspondant à la valeur de lumière reçue la plus récente.
